Europäisches Patentamt

European Patent Office

Office européen des brevets

(11) Numéro de publication : **0 421 830 B1**

(12) **FASCICULE DE BREVET EUROPEEN**

(45) Date de publication du fascicule du brevet :
**16.06.93 Bulletin 93/24**

(51) Int. Cl.$^5$ : **C07C 19/08, C07C 17/20**

(21) Numéro de dépôt : **90402407.2**

(22) Date de dépôt : **31.08.90**

(54) **Procédé de fabrication du 1,1,1-chlorodifluoroéthane.**

(30) Priorité : **03.10.89 FR 8912918**

(43) Date de publication de la demande :
**10.04.91 Bulletin 91/15**

(45) Mention de la délivrance du brevet :
**16.06.93 Bulletin 93/24**

(84) Etats contractants désignés :
**AT BE CH DE ES FR GB GR IT LI NL**

(56) Documents cités :
**EP-A- 0 297 947**
**FR-A- 2 337 120**
**CHEMICAL ABSTRACTS, vol. 82, 1975, page
468, résumé no. 111560v, Columbus, Ohio, US;
& JP-A-74 03 965**
**CHEMICAL ABSTRACTS, vol. 85, 1976, page
593, résumé no. 123323v, Columbus, Ohio, US;
& JP-A-76 29 404**

(73) Titulaire : **ELF ATOCHEM S.A.
4 & 8, Cours Michelet La Défense 10
F-92800 Puteaux (FR)**

(72) Inventeur : **Bergougnan, Michel
2 Rue du Docteur Roux
F-69310 Pierre-Bénite (FR)**
Inventeur : **Galland, Jean-Michel
1 Domaine des Essarts
F-69390 Vernaison (FR)**
Inventeur : **Perdrieux, Sylvain
707 Rue de la Maçonnière, Charly
F-69390 Vernaison (FR)**

(74) Mandataire : **Leboulenger, Jean et al
ATOCHEM Département Propriété Industrielle
Cédex 42- La Défense 10
F-92091 Paris la Défense (FR)**

EP 0 421 830 B1

## Description

La présente invention concerne un procédé de fabrication continue et sélective du 1,1,1-chlorodifluoroéthane par fluoration du 1,1,1-trichloroéthane avec de l'acide fluorhydrique anhydre en phase liquide et en présence d'un catalyseur.

Le 1,1,1-chlorodifluoroéthane (ci-après dénommé 142b) est utilisé comme matière première pour la fabrication du fluorure de vinylidène, comme propulseur dans l'industrie des aérosols, comme agent gonflant dans l'industrie des mousses et comme fluide de réfrigération.

La fabrication de 142b à partir de 1,1,1-trichloroéthane (ci-après dénommé T111) et d'acide fluorhydrique (HF), en particulier par les procédés dits "en phase liquide", avec ou sans catalyseur, est connue depuis longtemps.

La réaction procède de la manière simplifiée suivante :

$$CH_3CCl_3 (T111) + HF \rightarrow CH_3CCl_2F (141b) + HCl$$
$$CH_3CCl_2F (141b) + HF \rightarrow CH_3CClF_2 (142b) + HCl$$

Il se produit toutefois des réactions secondaires indésirables dans l'optique d'une production sélective de 142b. A titre d'exemple, la fluoration peut se poursuivre jusqu'au 1,1,1-trifluoroéthane (143a) :

$$CH_3CClF_2 (142b) + HF \rightarrow CH_3CF_3 (143a) + HCl$$

D'autres réactions parasites peuvent se produire selon les conditions réactionnelles, telles que des deshydrochlorations, des dimérisations etc..., suivies de fluorations ou de chlorations. Ces réactions parasites conduisent à des sous-produits organohalogénés qui n'appartiennent pas à la série dite "série 140" (T111, 141b, 142b, 143a).

Un premier objectif d'un procédé industriel de fabrication de 142b est de minimiser la production des sous-produits non désirés : 143a et produits n'appartenant pas à la série 140.

Un deuxième objectif d'un procédé industriel de fabrication de 142b est, au niveau de la réaction, de maximiser la conversion de l'acide fluorhydrique. Quel que soit le système réactionnel utilisé, une part de l'acide fluorhydrique mis en jeu n'est pas consommée et cet acide fluorhydrique doit être récupéré. Cette récupération est facilitée si le taux de transformation de l'acide fluorhydrique est élevé dans le système réactionnel. Il est connu (voir plus loin) que la fabrication de 142b à partir de T111 peut être effectuée en phase liquide sans catalyseur. Toutefois, dans ce cas, le taux de transformation de l'acide fluorhydrique dans le système réactionnel est normalement limité et la récupération de l'acide fluorhydrique non transformé nécessite l'utilisation de techniques lourdes en aval de la réaction. De ce fait, des catalyseurs ont été recherchés, permettant d'effectuer cette réaction avec un taux de transformation élevé de l'acide fluorhydrique et parallèlement avec des productivités plus importantes.

Un troisième objectif d'un procédé industriel de fabrication de 142b à partir de T111 est d'obtenir économiquement une qualité commerciale pour l'acide chlorhydrique sous-produit, permettant ainsi sa valorisation. Il est bien connu qu'un des moyens efficaces pour atteindre cet objectif est d'obtenir de l'acide chlorhydrique anhydre et purifié par distillation à l'aval du système réactionnel. Toutefois, cette distillation de l'acide chlorhydrique sousproduit nécessite, pour être économique, l'utilisation d'une pression absolue de l'ordre de 6 à 30 bars, plus précisément de l'ordre de 10 à 20 bars. L'utilisation d'une telle pression au niveau de la distillation de l'acide chlorhydrique anhydre implique, pour un procédé industriel, le fait de conduire la réaction de fluoration sous une pression au moins du même ordre, de manière à ce que les produits gazeux de la réaction (HCl et autres) transitent directement du système réactionnel à la distillation.

Ces divers objectifs, pour un procédé industriel de fabrication de 142b à partir de T111, apparaissent malheureusement contradictoires à l'homme de l'art. En effet, comme le montrera plus loin la revue des procédés existants, l'utilisation d'un catalyseur actif (permettant l'obtention d'un taux de transformation élevé de l'acide fluorhydrique) conduit normalement à une fluoration poussée de la série 140, donc à la sousproduction d'une fraction élevée de 143a indésirable, d'une part, et à un taux de sous-produits hors série 140 également élevé, d'autre part. De même, lorsque les conditions réactionnelles sont choisies de manière à minimiser le taux de 143a, le milieu réactionnel contient inévitablement des proportions élevées de T111 et 141b puisqu'il s'agit de réactions consécutives. Or, ces molécules peu ou pas fluorées sont les plus instables et, de ce fait, il se forme à priori des quantités plus importantes de sousproduits n'appartenant pas à la série 140. De même, le fait de choisir une pression relativement élevée pour conduire la réaction de fluoration (dans l'optique d'une récupération économique de l'acide chlorhydrique) favorise inévitablement, comme cela est bien connu en fluoration, la production des composés fluorés supérieurs et donc un taux de 143a élevé.

Aucun des procédés de fluoration du T111, connus à ce jour, ne permet d'atteindre simultanément les trois objectifs précités.

Le brevet FR 798 421 décrit succinctement la possibilité de produire du 141b, du 142b et du 143a à partir de T111 et d'acide fluorhydrique à 150°C sans catalyseur ou en présence de SbF₃. Ce brevet ne donne aucune indication sur les moyens d'obtenir sélectivement du 142b.

Dans l'article J. Am. Chem. Soc, 58,889-890 (1936), HENNE et RENOLL décrivent la possibilité de produire du 141b, du 142b et du 143a à partir de T111 et d'acide fluorhydrique en présence de $SbF_3$ et $SbF_3Cl_2$. Toutefois cet article ne mentionne pas de conditions opératoires permettant de limiter la production de 143a.

Dans l'article J. Soc. Chem. Ind. (London), 67, 331-333 (1948), BROWN et WHALLEY décrivent la possibilité de produire du 141b, du 142b et du 143a à partir de T111 et d'acide fluorhydrique sans catalyseur, à 144°C. Toutefois, cet article ne mentionne également pas les conditions opératoires permettant de limiter la production de 143a.

La publication de brevet JP 74/3965 décrit un procédé d'obtention sélective de 142b à partir de T111 et d'acide fluorhydrique en présence du catalyseur $SbCl_5$, caractérisé principalement en ce que :

a) la pression de réaction est comprise entre 0 et 3 $kg/cm^2$ effectifs,

b) la réaction est effectuée en une série de deux réacteurs, le premier formant essentiellement du 142b à partir de 141b avec un excès d'acide fluorhydrique, le second formant essentiellement du 141b à partir de T111.

Cette conception de système réactionnel permet effectivement d'atteindre un taux de conversion global élevé de l'acide fluorhydrique et apparemment un taux relativement faible de 143a (environ 1,5 % du 142b formé).

Toutefois, ce procédé est conduit à basse pression ; il est bien mentionné dans ce brevet que si la réaction était menée au-dessus d'une pression de 3 $kg/cm^2$ effectif, le taux de 143a serait augmenté notablement et qu'un choix judicieux de la température réactionnelle ne permettrait pas de compenser cet effet. Ce procédé ne permet donc pas une récupération optimale de l'acide chlorhydrique sous-produit. Par ailleurs, le procédé utilisé pour obtenir un taux de transformation élevé de l'acide fluorhydrique est complexe puisqu'il nécessite deux systèmes réactionnels en série. Enfin, ce brevet ne mentionne pas le taux de sous produits hors série 140 formé.

Le brevet US 3 833 676 décrit un procédé de fabrication de 142b à partir de T111 et d'acide fluorhydrique en l'absence de catalyseur, caractérisé principalement en ce que la réaction est conduite en présence d'un excès d'acide fluorhydrique. Il est évident que, dans ce cas, le taux de transformation de l'acide fluorhydrique dans le système réactionnel est limité.

La publication de brevet JP 76/29404 décrit un procédé de fabrication de 142b à partir de T111 et d'acide fluorhydrique en présence du catalyseur $MoCl_5$. Dans le texte de ce brevet, on explique les problèmes de sélectivité posés par cette réaction : si l'on n'utilise pas de catalyseur, le taux de transformation de l'acide fluorhydrique est limité ; si l'on utilise un catalyseur actif tel que $SbCl_5$, on forme des quantités importantes de 143a, en particulier sous pression, ou bien on limite la réaction à une part plus faible de 142b et simultanément le taux de transformation de l'acide fluorhydrique se dégrade. Ce brevet recommande l'utilisation du catalyseur $MoCl_5$ comme étant plus sélectif que $SbCl_5$ ; on n'y trouve cependant aucune recommandation de conditions opératoires précises ou d'exemple permettant la conduite continue d'une réaction utilisant $MoCl_5$ ou même $SbCl_5$ comme catalyseur, et permettant l'obtention simultanée de moins de 1 % de 143a, et d'un taux de transformation de l'acide fluorhydrique supérieur à 90 %.

La publication de brevet JP 76/39606 décrit l'utilisation d'oxydes métalliques tels que MoO3 comme catalyseurs de fluoration en phase liquide. L'exemple de la fluoration du T111 est cité. Comme dans le brevet ci-dessus, on ne décrit pas de conditions opératoires précises permettant d'obtenir simultanément un faible taux de 143a et un taux élevé de transformation de l'acide fluorhydrique.

Le brevet FR 2 337 120 décrit un procédé de fabrication de 142b et/ou de 143a à partir de T111 et d'acide fluorhydrique en présence du catalyseur $SbCl_5$. Le procédé est caractérisé principalement en ce que la réaction est conduite dans un solvant lourd et inerte. Ce brevet indique deux exemples où l'on note à la fois un faible taux de 143a et un taux élevé de transformation de l'acide fluorhydrique (exemples 17 et 22). On constate cependant que les pressions de réaction recommandées sont faibles (de 0 à 5 $kg/cm^2$ effectifs) et que les deux exemples cités ci-dessus sont conduits à 0 et 1 $kg/cm^2$. Il est évident que, si les pressions de réaction étaient plus élevées, les taux de 143a le seraient également. Par ailleurs, les températures de réaction recommandées sont faibles, de 10 à 65°C, en relation avec des pressions faibles et le brevet indique que des températures de réaction plus élevées conduiraient à la formation de sous-produits indésirables. L'utilisation d'un solvant lourd permet donc d'obtenir des taux variables de 143a, mais en aucun cas de minimiser la formation de celui-ci, en particulier sous une pression supérieure à 5 $kg/cm^2$ effectifs.

Dans un procédé continu de fluoration du T111 en phase liquide, le milieu réactionnel liquide comporte nécessairement une part de produits organohalogénés n'appartenant pas à la série 140, qui proviennent des impuretés de la matière première ou sont formés, par réactions secondaires, à partir des composés de la série 140 présents. Une part de ces produits ont des points d'ébullition relativement élevés. Sauf précaution particulière, la teneur en ces produits dans le milieu réactionnel peut donc être élevée.

Il a maintenant été trouvé qu'il existe une relation directe entre la teneur en ces produits organohalogénés dans le milieu réactionnel et la sous-production de 143a, cette dernière étant d'autant plus élevée que la teneur en sous-produits hors série 140 est élevée dans le milieu réactionnel, et qu'il faut donc contrôler la teneur du milieu réactionnel en produits n'appartenant pas à la série 140 à moins de 40 % poids, de préférence à moins de 10 % poids, de manière à minimiser la sous-production de 143a.

Correlativement, les teneurs en composés de la série 140 du milieu réactionnel sont élevées, en particulier celles de T111 et de 141b. Bien que la production de sous-produits indésirables à partir de ces éléments soit à priori facilitée, il a en outre été trouvé que, dans certaines conditions réactionnelles, cela n'était curieusement pas le cas.

En d'autres termes, sous réserve de contrôler la pression, la température, la teneur en catalyseur actif et la teneur en sous-produits hors série 140 dans les fourchettes indiquées plus loin, il est possible d'atteindre simultanément les trois objectifs précités.

Le procédé selon la présente invention pour la fabrication continue du 1,1,1-chlorodifluoroéthane à partir de 1,1,1-trichloroéthane et d'acide fluorhydrique par réaction en phase liquide en présence d'au moins un catalyseur de fluoration, est caractérisé par la combinaison des moyens suivants :

**a)** la pression absolue du système réactionnel est choisie entre 6 et 30 bars, de préférence entre 10 et 20 bars ;

**b)** la teneur totale en catalyseur(s) milieu réactionnel liquide, exprimée en pourcentage pondéral de métal, est comprise entre 0,05 et 10%, de préférence entre 0,1 et 5 % ;

**c)** la température de réaction est choisie entre 50 et 120°C, de préférence entre 70 et 100°C ; et

**d)** la teneur du milieu réactionnel liquide en sous-produits n'appartenant pas à la série 140 est réglée de manière à rester inférieure à 40 % en poids, de préférence inférieure à 10 % en poids.

En opérant dans ces conditions, il a été constaté que :

- le taux de 143a sous-produit était très faible, typiquement de l'ordre de ou inférieur à 1 % poids de la production de 142b,
- le taux de production des sous-produits n'appartenant pas à la série 140 était très faible, typiquement de l'ordre de ou inférieur à 1,5 % poids de la production de 142b,
- le taux de transformation de l'acide fluorhydrique en sortie du système réactionnel était élevé, typiquement supérieur à 90 %,

et que ces résultats étaient atteints malgré une pression de réaction élevée, permettant une purification par distillation sous forme anhydre de l'acide chlorhydrique sous-produit.

Le ou les catalyseurs de fluoration utilisés dans le procédé selon l'invention sont les catalyseurs actifs bien connus tels que les halogénures, oxydes et oxyhalogénures des éléments des groupes IVa et b, Va et b, VIa et b et VIII. Un exemple typique est le pentachlorure d'antimoine ou plus exactement les chlorofluorures d'antimoine pentavalent formés in situ par fluoration partielle de $SbCl_5$. Si nécessaire, l'activité des catalyseurs peut être maintenue par les moyens connus tels que, par exemple, une injection de chlore dans le cas des chlorures d'antimoine.

La teneur du milieu réactionnel liquide en sous-produits hors série 140, peut être ajustée par une évacuation des produits de la réaction à partir du système réactionnel sous deux formes : une évacuation sous forme gaz et une évacuation sous forme liquide.

Le procédé selon l'invention peut être mis en oeuvre dans un appareillage classique bien connu de l'homme de l'art. Il peut être constitué d'un seul réacteur alimenté, sous forme gazeuse ou liquide, par les matières premières (T111 et HF) et les recyclages et chauffé ou refroidi de manière adéquate. Il doit favoriser le contact entre les réactifs par une géométrie, un mode d'introduction des réactifs et une technique de mélangeage appropriés. Ce réacteur peut être surmonté d'une colonne et d'un condenseur de rétrogradation permettant d'éviter un départ, dans le flux gazeux issu du réacteur, du ou des catalyseurs utilisés et d'ajuster la composition en organofluorés de ce flux (teneur en 142b, 141b, T111, etc...).

Les matières premières ou recyclats (T111, 141b, HF) sont alimentés au réacteur dans le rapport adéquat pour une production de 142b. Cela signifie, dans le cas d'un recyclage complet des produits non convertis (HF, T111, 141b), un rapport molaire HF frais/T111 frais voisin de la stoechiométrie, c'est-à-dire environ 2.

Comme indiqué ci-dessus, l'appareillage réactionnel peut comporter une sortie liquide des produits de la réaction. Cette sortie sous forme liquide est un des moyens possibles permettant d'ajuster la teneur du milieu réactionnel en produits n'appartenant pas à la série 140 .

Les flux issus de la réaction, gazeux et éventuellement liquides, sont traités de manière conventionnelle pour en séparer les produits finis utiles (142b, HCl). Ce traitement comporte notamment, pour ce qui est de la récupération de l'acide chlorhydrique, une distillation de HCl anhydre. Le T111 et le 141b non transformés, l'acide fluorhydrique non transformé en faible quantité, et le ou les catalyseurs utilisés contenus dans la sortie liquide du réacteur sont recyclés au système réactionnel.

Les exemples 1 et 2 suivants illustrent l'invention, sans la limiter. Les exemples 3 et 4 sont donnés à titre de comparaison pour mettre en évidence l'intérêt des paramètres de fonctionnement préconisés selon l'invention.

## EXEMPLE 1

On utilise un réacteur conventionnel de fluoration de 5m³, en acier ordinaire, surmonté d'une colonne de rétrogradation et d'un condenseur de reflux. Ce réacteur est muni d'alimentations continues en T111 frais et recyclé, 141b recyclé, HF frais et recyclé, chlore et pentachlorure d'antimoine.

La pression de réaction est régulée à 11 bars absolus. La température du milieu réactionnel est régulée à 79°C par chauffage du réacteur au travers d'une double enveloppe.

L'adjonction de pentachlorure d'antimoine est réglée de manière à obtenir une teneur en antimoine (comptée en Sb métal) de 1 % poids dans le milieu réactionnel.

Un soutirage sous forme gazeuse est effectué à l'aval du condenseur de reflux situé en tête de la colonne surmontant le réacteur. Un soutirage sous forme liquide est effectué à partir du réacteur lui-même et réglé de manière à obtenir 4,3 % poids de sous-produits hors série 140 dans le milieu réactionnel.

Ces deux soutirages sont dirigés vers les dispositifs conventionnels de traitement pour la récupération ou le recyclage des produits.

Le bilan matière, indiqué dans le tableau 1 suivant, montre l'intérêt de ces conditions de fonctionnement. On peut constater les performances simultanées suivantes :

**a)** pression de marche : 11 bars abs,

**b)** taux de transformation de l'acide fluorhydrique : 90,5 %

**c)** taux de 143a formé par rapport à la production de 142b : $\dfrac{143a}{142b} = \dfrac{6,1}{915,5} = 0,5$ % poids

**d)** taux de sous-produits (SP) hors série 140 formés par rapport à la production de 142b : $\dfrac{SP}{142b} = \dfrac{12,9}{915,5} = 1,4$ % poids

**TABLEAU 1**
**BILAN MATIERE Kg/h**

| Produits | Alimentation ou recyclage | Soutirage gaz (sortie condenseur) | Soutirage liquide (sortie réacteur) |
|---|---|---|---|
| HCl | – | 676,4 | $\epsilon$ |
| HF | 407,9 (frais+recyclé) | 38,6 | $\epsilon$ |
| 143a | – | 6,1 | $\epsilon$ |
| 142b | – | 860 | 55,5 |
| 141b | 234,6 (recyclé) | 35,1 | 199,5 |
| T111 | 1267,9 (frais+recyclé) | $\epsilon$ | 32,1 |
| $Cl_2$ | 5,8 | $\epsilon$ | $\epsilon$ |
| Sb (1) | 3 (recyclé) | – | 3 |
| SP (2) | – | – | 12,9 |

(1) Exprimé en antimoine métal

(2) Sous-produits organohalogénés hors série 140

## EXEMPLE 2

On utilise le même réacteur que dans l'exemple 1.

La pression de réaction est régulée à 16 bars abs. La température du milieu réactionnel est régulée à 75°C.

L'adjonction de pentachlorure d'antimoine est réglée de manière à obtenir une teneur en antimoine du milieu réactionnel (comptée en Sb métal) de 1,1 % poids.

Le soutirage liquide du réacteur est réglé de manière à obtenir 3 % poids de sous-produits hors série 140 dans le milieu réactionnel.

Le bilan matière, indiqué dans le tableau 2, permet de constater les performances simultanées suivantes :

a) pression de marche : 16 bars abs,

b) taux de transformation de l'acide fluorhydrique : 91,2 %

c) taux de 143a formé par rapport à la production de 142b : $\dfrac{143a}{142b} = \dfrac{4,6}{480} = 1$ % poids

**d)** taux de sous-produits hors série 140 formés par rapport à la production de 142b : $\dfrac{SP}{142b} = \dfrac{5,9}{480} = 1,2$

% poids

<div align="center">

**TABLEAU 2**

**BILAN MATIERE Kg/h**

</div>

| Produits | Alimentation ou recyclage | Soutirage gaz (sortie condenseur) | Soutirage liquide (sortie réacteur) |
|---|---|---|---|
| HCl | - | 356 | $\epsilon$ |
| HF | 213,3 (frais+recyclé) | 18,8 | $\epsilon$ |
| 143a | - | 4,6 | $\epsilon$ |
| 142b | - | 421,4 | 58,6 |
| 141b | 167,3 (recyclé) | 86,3 | 81,0 |
| T111 | 688,9 (frais+recyclé) | $\epsilon$ | 39,5 |
| $Cl_2$ | 2,6 | $\epsilon$ | $\epsilon$ |
| Sb (1) | 2,1 (recyclé) | - | 2,1 |
| SP (2) | - | - | 5,9 |

(1) exprimé en antimoine métal

(2) sous-produits organohalogénés hors série 140

## EXEMPLE COMPARATIF 3

On utilise le même réacteur que dans l'exemple 1. La pression est régulée à 11 bars abs et la température du milieu réactionnel à 75°C.

L'adjonction de pentachlorure d'antimoine est réglée de manière à obtenir une teneur en antimoine (comptée en Sb métal) de 1,3 % poids du milieu réactionnel et le soutirage liquide du réacteur est réglé de manière à obtenir 52 % poids de sous-produits hors série 140 dans le milieu réactionnel (soit une valeur supérieure au seuil recommandé selon l'invention).

<div align="center">

7

</div>

Le bilan matière pour un tel fonctionnement est indiqué dans le tableau 3. On obtient les performances suivantes :

**a)** pression de marche : 11 bars abs,

**b)** taux de transformation de l'acide fluorhydrique : 90,4 %

**c)** taux de 143a formé par rapport à la production de 142b : $\dfrac{143a}{142b} = \dfrac{19,9}{410,5} = 4,9$ % poids

**d)** taux de sous-produits hors série 140 formés par rapport à la production de 142b : $\dfrac{SP}{142b} = \dfrac{5,3}{410,5} = 1,3$ % poids

Par comparaison avec les exemples 1 et 2, on constate que le taux de 143a indésirable formé est environ 5 fois supérieur.

## TABLEAU 3

### BILAN MATIERE Kg/h

| Produits | Alimentation ou recyclage | Soutirage gaz (sortie condenseur) | Soutirage liquide (sortie réacteur) |
|---|---|---|---|
| HCl | – | 325,7 | $\epsilon$ |
| HF | 196,7 (frais+recyclé) | 18,8 | $\epsilon$ |
| 143a | – | 19,9 | $\epsilon$ |
| 142b | – | 407,7 | 2,8 |
| 141b | 7,6 (recyclé) | 5,4 | 2,2 |
| T111 | 581,5 (frais+recyclé) | $\epsilon$ | 0,7 |
| $Cl_2$ | 2,7 | $\epsilon$ | $\epsilon$ |
| Sb (1) | 0,14 (recyclé) | – | 0,14 |
| SP (2) | – | – | 5,3 |

(1) exprimé en antimoine métal

(2) sous-produits organohalogénés hors série 140

## EXEMPLE COMPARATIF 4

Dans cet exemple, on effectue une marche à basse pression, au-dessous du seuil recommandé selon l'invention. On conserve une température de réaction et une teneur en catalyseur voisines de celles des exemples 1 et 2. Dans ces conditions, de manière à obtenir une marche stable, il s'avère que l'on ne peut pas notablement abaisser la teneur en sous-produits hors série 140 du milieu réactionnel et cette teneur est donc nécessairement supérieure au seuil recommandé.

On utilise le même réacteur que dans l'exemple 1. La pression est régulée à 4,5 bars abs et la température du milieu réactionnel est régulée à 75°C.

L'adjonction de pentachlorure d'antimoine est ajustée de manière à obtenir une teneur en antimoine dans le milieu réactionnel (comptée en Sb métal) de 1,3 poids.

Le soutirage liquide du réacteur est réglé de manière à obtenir 93 % poids de sous produits hors série 140 dans le milieu réactionnel.

Le bilan matière, indiqué dans le tableau 4, permet d'établir les performances suivantes d'un tel fonctionnement :

**a)** pression de marche : 4,5 bars abs

**b)** taux de transformation de l'acide fluorhydrique : 92,5 %

**c)** taux de 143a formé par rapport à la production de 142b : $\dfrac{143a}{142b} = \dfrac{38,6}{375,3} = 10,3$ % poids

**d)** taux de sous-produits hors série 140 formés par rapport à la production de 142b : $\dfrac{SP}{142b} = \dfrac{9,3}{375,3} = 2,5$ % poids

On constate que ce mode de fonctionnement à une pression ne permettant pas une récupération aisée de l'acide chlorhydrique formé conduit en outre à des taux de 143a et de sous-produits hors série 140 très supérieurs à ceux observés dans les exemples 1 et 2 selon l'invention.

**TABLEAU 4**

**BILAN MATIERE Kg/h**

| Produits | Alimentation | Soutirage gaz | Soutirage liquide |
|----------|--------------|---------------|-------------------|
| HCl | - | 326,9 | $\epsilon$ |
| HF | 192,4 (frais+recyclé) | 14,6 | $\epsilon$ |
| 143a | - | 38,6 | $\epsilon$ |
| 142b | - | 375,1 | 0,2 |
| 141b | 15,5 (recyclé) | 15,3 | 0,2 |
| T111 | 567,7 (frais+recyclé) | $\epsilon$ | 0,3 |
| $Cl_2$ | 4,9 | $\epsilon$ | $\epsilon$ |
| Sb (1) | 0,13 (recyclé) | - | 0,13 |
| SP (2) | - | - | 9,3 |

(1) exprimé en antimoine métal

(2) sous-produits organohalogénés n'appartenant pas
à la série 140

**Revendications**

1. Procédé de fabrication continue du 1,1,1-chlorodifluoroéthane à partir de 1,1,1-trichloroéthane et d'acide fluorhydrique par réaction en phase liquide en présence d'au moins un catalyseur de fluoration caractérisé en ce que :

a) la réaction est conduite sous une pression absolue comprise entre 6 et 30 bars;

b) la teneur en catalyseur(s) du milieu réactionnel liquide, exprimée en pourcentage pondéral de métal, est comprise entre 0,05 et 10% ;

c) la température de réaction est choisie entre 50 et 120°C ; et

d) la teneur du milieu réactionnel liquide en sous-produits organohalogénés autres que le 1,1,1-trichloroéthane, le 1,1,1-dichlorofluororéthane, le 1,1,1-chlorodifluoroéthane et le 1,1,1-trifluoroéthane est réglée à moins de 40 % en poids.

2.  Procédé selon la revendication 1, caractérisé en ce que la réaction est conduite sous une pression absolue comprise entre 10 et 20 bars.

3.  Procédé selon la revendication 1 ou 2, caractérisé en ce que le ou les catalyseurs de fluoration sont choisis parmi les halogénures, oxydes et/ou oxyhalogénures des éléments des groupes IVa et b, Va et b, VIa et b et VIII.

4.  Procédé selon la revendication 3, caractérisé en ce que la réaction est conduite en présence de chlorofluorures d'antimoine comme catalyseur.

5.  Procédé selon l'une des revendications 1 à 4, caractérisé en ce que la teneur en catalyseur(s) du milieu réactionnel est comprise entre 0,1 et 5 % poids de métal.

6.  Procédé selon l'une des revendications 1 à 5, caractérisé en ce que la température de réaction est comprise entre 70 et 100°C.

7.  Procédé selon l'une des revendications 1 à 6, caractérisé en ce que la teneur du milieu réactionnel liquide en sous-produits organohalogénés est réglée à moins de 10 % en poids.

8.  Procédé selon l'une des revendications 1 à 7, caractérisé en ce que la teneur en sous-produits organohalogénés dans le milieu réactionnel liquide est réglée par ajustement relatif des évacuations sous forme gazeuse et liquide des produits de la réaction à partir du système réactionnel.

## Patentansprüche

1.  Verfahren zur kontinuierlichen Herstellung von 1,1,1-Chlordifluorethan ausgehend von 1,1,1-Trichlorethan und Fluorwasserstoffsäure durch Reaktion in der flüssigen Phase in Gegenwart von mindestens einem Fluorierungskatalysator dadurch gekennzeichnet, daß:
    a) die Reaktion bei einem absoluten Druck zwischen 6 und 30 bar durchgeführt wird;
    b) der Gehalt des flüssigen Reaktionsgemisches an Katalysator(en), ausgedrückt in Gewichtsprozenten des Metalls, zwischen 0.5% und 10% liegt;
    c) die Reaktionstemperatur zwischen 50 und 120°C gewählt wird; und
    d) der Gehalt des flüssigen Reaktionsgemisches an anderen organohalogenierten Nebenprodukten als 1,1,1-Trichlorethan, 1,1,1-Dichlorfluorethan, 1,1,1-Chlordifluorethan und 1,1,1-Trifluorethan auf weniger als 40 Gewichtsprozente eingestellt ist.

2.  Verfahren nach Anspruch 1, dadurch gekennzeichnet, daß die Reaktion unter einem absoluten Druck, der zwischen 10 und 20 bar liegt, durchgeführt wird.

3.  Verfahren nach Anspruch 1 oder 2, dadurch gekennzeichnet, daß der oder die Fluorierungskatalysator(en) aus den Halogeniden, Oxiden und/oder Oxohalogeniden der Elemente der Gruppen IVa und b, Va und b, VIa und b und VIII ausgewählt werden.

4.  Verfahren nach Anspruch 3, dadurch gekennzeichnet, daß die Reaktion in Gegenwart von Chlorofluoriden des Antimons als Katalysator durchgeführt wird.

5.  Verfahren nach einem der Ansprüche 1 bis 4, dadurch gekennzeichnet, daß der Katalysator(en)gehalt des Reaktionsgemisches zwischen 0,1 und 5 Gewichtsprozenten des Metalls beträgt.

6.  Verfahren nach einem der Ansprüche 1 bis 5, dadurch gekennzeichnet, daß die Reaktionstemperatur zwischen 70 und 100°C liegt.

7.  Verfahren nach einem der Ansprüche 1 bis 6, dadurch gekennzeichnet, daß der Gehalt des flüssigen Reaktionsgemisches an organohalogenierten Nebenprodukten auf weniger als 10 Gewichtsprozent eingestellt ist.

8.  Verfahren nach einem der Ansprüche 1 bis 7, dadurch gekennzeichnet, daß der Gehalt des flüssigen Reaktionsgemisches an organohalogenierten Nebenprodukten durch aufeinander abgestimmte Entnahmen von gasförmigen oder flüssigen Reaktionsprodukten aus dem Reaktionssystem eingestellt wird.

## Claims

1. Process for the continuous manufacture of 1,1,1-chlorodifluoroethane from 1,1,1-trichloroethane and hydrofluoric acid by reaction in the liquid phase in the presence of a least one fluorination catalyst, characterised in that:
   a) the reaction is carried out under an absolute pressure of between 6 and 30 bars;
   b) the content of catalyst(s) in the liquid reaction mixture, expressed in percent by weight of metal, is between 0.05 and 10 %;
   c) the reaction temperature is chosen between 50 and 120°C; and
   d) the content in the liquid reaction mixture of organohalogenated by-products, other than 1,1,1-trichloroethane, 1,1,1-dichlorofluoroethane, 1,1,1-chlorodifluoroethane and 1,1,1-trifluoroethane, is adjusted to less than 40 % by weight.

2. Process according to Claim 1, characterised in that the reaction is carried out under an absolute pressure of between 10 and 20 bars.

3. Process according to Claim 1 or 2, characterised in that the fluorination catalyst(s) are chosen from the halides, oxides and/or oxyhalides of the elements from groups IVa and b, Va and b, VIa and b and VIII.

4. Process according to Claim 3, characterised in that the reaction is carried out in the presence of antimony chlorofluorides as catalyst.

5. Process according to one of Claims 1 to 4, characterised in that the content of catalyst(s) in the reaction mixture is between 0.1 and 5 % by weight of metal.

6. Process according to one of Claims 1 to 5, characterised in that the reaction temperature is between 70 and 100°C.

7. Process according to one of Claims 1 to 6, characterised in that the content of organohalogenated by-products in the liquid reaction mixture is adjusted to less than 10 % by weight.

8. Process according to one of Claims 1 to 7, characterised in that the content of organohalogenated by-products in the liquid reaction mixture is adjusted by relative adjustement of the discharges from the reaction system, in the gaseous and liquid form, of the products of the reaction.